# EUROPÄISCHE PATENTSCHRIFT

(11) **EP 3 590 549 B1**
(45) Veröffentlichungstag und Bekanntmachung des Hinweises auf die Patenterteilung: **07.09.2022**
(21) Anmeldenummer: 19191782.2
(22) Anmeldetag: 13.02.2008
(51) Int. Cl.: A61L 27/22, A61L 27/50, A61L 27/54, A61L 27/30

(54) **VERFAHREN ZUR HERSTELLUNG VON IMPLANTATEN MIT EINER ULTRAHYDROPHILEN OBERFLÄCHE**
METHOD FOR THE PRODUCTION OF IMPLANTS WITH AN ULTRAHYDROPHILIC SURFACE
PROCÉDÉ DE FABRICATION D'IMPLANTS DOTÉS D'UNE SURFACE ULTRAHYDROPHILE

(30) Priorität: 14.02.2007 DE 102007007865
(43) Veröffentlichungstag der Anmeldung: 08.01.2020
(62) Teilanmeldung aus: 11159039.4
(73) Patentinhaber: Nobel Biocare Services AG, 8302 Kloten (CH)
(72) Erfinder: Lüers, Steffen, 45147 Essen (DE); Laub, Markus, 45134 Essen (DE); Jennissen, Herbert, 50858 Köln (DE)
(74) Vertreter: Nobbe, Matthias

(56) Entgegenhaltungen:
- EP-A1- 1 847 278
- M. CHATZINIKOLAIDOU ET AL.: "Stability of Surface-Enhanced Ultrahydrophilic Metals as a Basis for Bioactive rhBMP-2 Surfaces", MATERIALWISSENSCHAFT UND WERKSTOFFTECHNIK, VCH VERLAGSGESELLSCHAFT, WEINHEIM, DE, Bd. 34, Nr. 12, 2003, Seiten 1106-1112, XP002527659,

## Beschreibung

Die vorliegende Erfindung betrifft ein Verfahren zur Herstellung von lagerfähigen Implantaten mit einer ultrahydrophilen Oberfläche sowie die so hergestellten Implantate. Es werden auch Verfahren zur Herstellung beladener, so genannter bioaktiver Implantatoberflächen von metallischen oder keramischen Materialien, die für Implantate wie künstliche Knochen, Gelenke, Zahnimplantate oder auch Kleinstimplantate, z.B. sogenannte Stents, verwendet werden, als auch nach den Verfahren weiter hergestellte Implantate, die als sogenannte "delivery devices" eine kontrollierte Freisetzung, z.B. über Dissoziation, der bioaktiven Moleküle von den Implantatmaterialien erlauben, beschrieben, die nicht Gegenstand der vorliegenden Anmeldung sind.

Die Implantation künstlicher Gelenke oder Knochen hat in den letzen Jahren eine zunehmende Bedeutung z.B. bei der Behandlung von Gelenkdysplasien oder -luxationen bzw. bei Erkrankungen, die auf der Abnutzung von Gelenken als Folge von Gelenkfehlstellungen entstehen können, gewonnen. Die Funktion der Implantate und die für ihre Herstellung verwendeten Materialien, die neben Metallen wie Titan oder Metalllegierungen auch Keramik oder Kunststoffmaterialien wie Teflon oder Polylactiden umfassen können, sind stetig verbessert worden, so dass Implantate nach erfolgreichem Heilungsverlauf in 90-95% der Fälle Standzeiten von 10 Jahren aufweisen können.

Ungeachtet dieser Fortschritte und verbesserter operativer Verfahren bleibt eine Implantation immer noch ein schwieriger und belastender Eingriff, insbesondere da sie mit einem langwierigen Einheilungsprozess des Implantates, der oft monatelange Klinik- und Kuraufenthalte einschließlich Rehabilitationsmaßnahmen umfasst, verbunden ist. Neben den Schmerzen stellen dabei für die betroffenen Patienten die Länge der Behandlungsdauer und die Trennung von der vertrauten Umgebung große Belastungen dar. Ferner verursacht der langwierige Heilungsprozess durch die erforderliche intensive Betreuung hohe Personal- und Pflegekosten.

Das Verständnis der Vorgänge auf der molekularen Ebene, die für ein erfolgreiches Einwachsen eines Implantates erforderlich sind, hat sich in den letzten Jahren bedeutend erweitert. Entscheidend für die Gewebeverträglichkeit eines Implantates sind Strukturkompatibilität und Oberflächenkompatibilität. Die Biokompatibilität im engeren Sinne ist alleine von der Oberfläche bedingt. Auf allen Ebenen der Integration spielen Proteine eine maßgebliche Rolle. Wie nachfolgend erläutert, entscheiden sie bereits während der Implantationsoperation durch die Ausbildung einer initialen adsorbierten Proteinschicht über den weiteren Verlauf der Implantateinheilung, da sich auf dieser Schicht später die ersten Zellen ansiedeln.

Bei der molekularen Interaktion zwischen Implantat, das auch als Biomaterial bezeichnet wird, und Gewebe, findet eine Vielzahl von Reaktionen statt, die streng hierarchisch geordnet zu sein scheinen. Als erste biologische Reaktion findet die Adsorption von Proteinen an der Oberfläche des Biomaterials statt. In der dadurch entstandenen Proteinschicht werden anschließend einzelne Proteinmoleküle beispielsweise durch Konformationsänderungen zu Signalstoffen, die auf der Oberfläche präsentiert werden, umgewandelt oder durch katalytische (proteolytische) Reaktionen werden als Signalstoffe wirkende Proteinfragmente freigesetzt.

Ausgelöst durch die Signalstoffe findet in der nächsten Phase die zelluläre Besiedlung statt, die eine Vielzahl von Zellen wie Leukozyten, Makrophagen, Immunozyten, und schließlich auch Gewebezellen (Fibroblasten, Fibrozysten, Osteoblasten, Osteozyten) umfassen kann. In dieser Phase spielen andere Signalstoffe, sogenannte Mediatoren, wie z.B. Cytokine, Chemokine, Morphogene, Gewebshormone und echte Hormone eine entscheidende Rolle. Im Falle einer Biokompatibilität kommt es schließlich zur Integration des Implantates in den Gesamtorganismus, und idealerweise erhält man ein Permanentimplantat.

Im Lichte von Arbeiten, die in den letzten Jahren auf molekularer Ebene der Osteogenese durchgeführt worden sind, haben chemische Signalstoffe, die sogenannten "bone morphogenic Proteins" (BMP-1-BMP-15), die Einfluss auf das Knochenwachstum besitzen, eine zunehmende Bedeutung gewonnen. BMPs (insbesondere BMP-2 und BMP-4, BMP-5, BMP-6, BMP-7) sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von alkalischer Phosphatase, Hormonrezeptoren, knochenspezifischer Substanzen wie Kollagen Typ 1, Osteocalcin, Osteopontin und schließlich die Mineralisation.

Die BMP-Moleküle regulieren dabei die drei Schlüsselreaktionen Chemotaxis, Mitose und Differenzierung der jeweiligen Vorläuferzelle. Darüber hinaus spielen BMPs eine wichtige Rolle in der Embryogenese, Organogenese des Knochens und anderer Gewebe, wobei als Zielzellen Osteoblasten, Chondroblasten, Myoblasten und vaskulare glatte Muskelzellen (Proliferationshemmung durch BMP-2) bekannt sind.

Inzwischen sind 15 BMPs inklusive multipler Isoformen bekannt. Bis auf das BMP-1 gehören die BMPs der "transforming growth factor beta" (TGF-ß) Superfamilie an, für die spezifische Rezeptoren auf den Oberflächen der entsprechenden Zellen nachgewiesen wurden. Wie der erfolgreiche Einsatz von rekombinanten humanen BMP-2 und/oder BMP-7 in Versuchen zu Defektheilungsprozessen an Ratten, Hunden, Kaninchen und Affen gezeigt hat, scheint keine Speziesspezifität vorzuliegen.

Bisherige Versuche, die die Knochenbildung auslösenden Eigenschaften der BMPs gezielt für Implantationszwecke auszunutzen, indem das BMP-2 und/oder BMP-7 direkt auf metallische oder keramische Biomaterialien aufgebracht wurden, sind jedoch weitestgehend erfolglos verlaufen.

Im Stand der Technik ist eine Reihe von Arbeiten auf dem Gebiet beschichteter Implantatmaterialien bekannt. So beschreibt die WO9926674 ein Verfahren zur Herstellung bioaktiver Implantatoberflächen von metallischen oder keramischen Materialien, bei dem in einem ersten Schritt Ankermoleküle an die Oberfläche des Implantatmaterials kovalent gebunden werden und in einem zweiten Schritt Peptide kovalent an die Ankermoleküle gebunden werden.

In der WO0209788 wird ein Verfahren zur Herstellung bioaktiver Implantatoberflächen von metallischen oder keramischen Materialien bereitgestellt, bei dem in einem ersten Schritt Ankermoleküle mit hydrophoben Resten an der Oberfläche des Implantatmaterials kovalent gebunden werden und in einem zweiten Schritt Peptide auf das so behandelte Implantatmaterial gegeben werden, die infolge nicht-kovalenter Wechselwirkungen zwischen den Peptiden und den hydrophoben Resten der Ankermoleküle immobilisiert werden.

Gemäß diesen beiden Dokumenten ist es somit erforderlich, auf der Oberfläche des Implantates Ankermoleküle chemisch zu immobilisieren, die dann mit den Peptiden kovalent chemisch verbunden werden oder infolge nicht-kovalenter Wechselwirkungen auf der Implantatoberfläche gebunden werden. Versuchsergebnisse der Erfinder haben dabei gezeigt, dass Versuche zur Immobilisierung von Peptiden auf der Implantatoberfläche ohne Ankermoleküle nicht erfolgreich waren.

M. Chatzinikolaidou et al., Mat.-wiss. u. Werkstofftech. 2003, 34, No. 12, 1106-1112, offenbart die Oxidation von Implantaten mit Chromschwefelsäure bei hohen Temperaturen zur Herstellung von ultrahydrophilen Oberflächen.

EP 1 847 278 A offenbart ein Verfahren bei dem eine hydrophile Oberfläche mit einer Schutzschicht aus Salz versehen wird.

Seitens der Erfinder wurde nun überraschend, insbesondere im Hinblick auf diese früheren Versuche der Erfinder zur Immobilisierung, gefunden, dass eine Immobilisierung von Peptiden auf Metalloberflächen, insbesondere von Wachstumsfaktoren der TGF Klasse, z.B. BMP-Proteine, erreicht werden kann, wenn eine ausreichend hydrophile Oberfläche auf dem Implantatmaterial bereitgestellt werden kann. Seitens der Erfinder wurde gefunden, dass dieses erreicht werden kann, wenn man durch Behandlung mit einem Oxidationsmittel eine ultrahydrophile Oxidschicht auf der Metalloberfläche erzeugt.

Die Erfinder haben festgestellt, dass Oberflächen mit einer hohen Oberflächenenergie eine starke Gewebe-Bioadhäsion aufweisen können. Da Oberflächen mit einer hohen Oberflächenenergie zumeist niedrige Kontaktwinkel mit Wasser aufweisen, lässt sich eine solche Oberfläche über die Messung von dynamischen Kontaktwinkeln sehr leicht identifizieren. Kleine Kontaktwinkel kennzeichnen eine hohe Benetzbarkeit einer Oberfläche.

Bei den dynamischen Kontaktwinkeln unterscheidet man einen Vorrückwinkel (θᵥₒᵣ) von einem Rückzugswinkel (θ_{rück}) und bezeichnet die Differenz dieser Winkel als Kontaktwinkelhysterese. Dabei ist der Vorrückwinkel charakteristisch für die Hydrophilizitäts-Hydrophobizitäts-Eigenschaften einer Oberfläche und entspricht weitgehend dem sog. statischen Kontaktwinkel. Je größer die Hysterese, desto größer ist in der Regel die Heterogenität der Oberfläche. Mechanisch polierte oder elektro-polierte Titanoberflächen haben normalerweise dynamische Kontaktwinkel (Vorrückwinkel) von 70-80° und besitzen gemäß einschlägiger Literatur eine geringere Gewebe-Bioadhäsion. Daher ist es gemäß der Entwicklung der Erfinder erstrebenswert, auch auf Metallen Oberflächen mit niedrigen Kontaktwinkeln bereitzustellen.

Oberflächen mit dynamischen Kontaktwinkeln von 0 bis 10° werden erfindungsgemäß als "ultrahydrophil" definiert. Sie weisen gleichzeitig eine charakteristische Nanostruktur auf. In tierexperimentellen Arbeiten konnte seitens der Erfinder gezeigt werden, daß die Knochendichte nach 4 Wochen in der Umgebung eines ultrahydrophilen Implantates doppelt so hoch ist wie in Umgebung des Kontroll-Implantates.

Zwar wurden im Stand der Technik gemäß EP 1 150 620 bereits Implantate mit hydrophilen Oberflächen nach Sandstrahlung und Säureätzung beschrieben, auf denen Benetzungswinkel mit Wasser zwischen 20-50° gemessen wurden. Solche Oberflächen wurden als "hydrophil" bezeichnet und konnten in bestimmten Salzlösungen konserviert werden. Allerdings wurde gemäß der EP 1 150 620 beobachtet, dass derartige Oberflächen steigenden Salzkonzentration gegenüber empfindlich waren.

Seit vielen Jahren ist im Stand der Technik weiterhin bekannt, daß hydrophile Metalloberflächen, beispielsweise aus Titan, nicht stabil sind, sondern spontan wieder hydrophob werden. Der chemische Zustand der Oberfläche von Titan und Titanbasislegierungen ist komplex. Es wird davon ausgegangen, dass die Oberfläche von Titanmetall in Luft und Wasser spontan oxidiert und dass dann an der Oberfläche, das heißt in der äußersten Atomschicht des Oxids, eine Reaktion mit Wasser stattfindet, wobei Hydroxylgruppen gebildet werden.

Entsprechend sind solche Oberflächen besonders sensibel gegenüber einer Gammasterilisierung, einer Methode, die heute weit verbreitet in der Herstellung klinisch einsetzbarer Implantate verwendet wird. So wurde im Stand der Technik gezeigt, daß Titandioxidschichten durch Lichteinstrahlung hydrophil gemacht werden können. Auch diese Schichten verlieren ihre Hydrophilizität bereits nach kurzer Zeit und werden wieder hydrophob, wobei der exakte Mechanismus dieser Veränderung immer noch unklar ist.

Es besteht demnach ein Bedarf an einem Verfahren, welches die Herstellung von Implantaten erlaubt, die ultrahydrophilen Schichten darauf unbegrenzt stabilisiert aufweisen und gleichzeitig einer Sterilisierung standhalten.

Die Erfindung ist auf ein Verfahren zur Herstellung eines lagerfähigen Implantates mit einer ultrahydrophilen Oberfläche gerichtet, bei dem man das Implantat mit einer ultrahydrophilen Oberfläche mit dynamischen Kontaktwinkeln von 0° bis 10° bei Benetzung der Oberfläche des Implantates mit Wasser und mit einer Kontaktwinkelhysterese von weniger als 5° bei Benetzung der Oberfläche des Implantates mit Wasser in eine salzhaltige wässrige Lösung gibt, die gegenüber der Oberfläche inert ist und die das Implantat allseits umschließt, und man die salzhaltige wässrige Lösung unter Bildung einer Exsikkationsschicht, die die Oberfläche des Implantats stabilisiert und schützt, evaporiert, wobei das Implantat aus einem Material, das aus der Gruppe der Metalle, der metallischen Legierungen und Kombinationen davon mit keramischen Materialien ausgewählt wird, besteht und das optional eine Oxidschicht auf der Oberfläche aufweist.

Die Erfindung bezieht sich auch auf ein lagerfähiges Implantat, das nach dem oben genannten Verfahren erhältlich ist oder das eine ultrahydrophile Oberfläche, die mit einer Exsikkationsschicht beschichtet ist, aufweist.

Die Erfinder beschreiben hier auch ein Verfahren zur Herstellung eines Implantates mit einer ultrahydrophilen Oberfläche, bei dem man die Oberfläche des Implantates so lange mit einem Oxidationsmittel behandelt, um auf der Oberfläche des Metalls eine Oxidschicht zu erzielen, bis sich eine Kontaktwinkelhysterese von weniger als 5° bei Benetzung der Oberfläche des Implantates mit Wasser ergibt, wobei das Implantat aus einem Material, das aus der Gruppe der Metalle, der metallischen Legierungen und Kombinationen davon mit keramischen Materialien ausgewählt wird, besteht.

Dabei besteht das Metallimplantat bevorzugt aus einem Material, das aus der

Gruppe der Metalle, der metallischen Legierungen und Kombinationen davon mit keramischen Materialien ausgewählt wird. Bevorzugt besteht das eingesetzte Implantatmaterial aus metallischen Materialien wie Reintitan oder metallischen Titanlegierungen, Chrom/Nickel/Aluminium/Vanadium/Kobalt-Legierungen (z.B. TiAIV4, TiAIFe2,5), Edelstählen (z.B. V2A, V4A, Chrom-Nickel 316L) oder aus einer Kombination davon mit keramischen Materialien wie Hydroxyapatit, Aluminiumoxid, bei der das metallische Material als Verbundmaterial mit keramischem Material vorliegt.

Mit Hilfe des Verfahrens lassen sich somit ultrahydrophile Metalloberflächen herstellen. Dazu behandelt man die Oberfläche des Metallimplantates so lange mit einem Oxidationsmittel, um auf der Oberfläche des Metalls eine Oxidschicht zu erzielen, bis sich eine Kontaktwinkelhysterese von weniger als 5°, bevorzugter von weniger als 1°, besonders bevorzugt von weniger als 0,5° bei Benetzung mit reinem (destilliertem) Wasser ergibt.

Bevorzugt dauert diese Behandlung solange, bis die Kontaktwinkelhysterese im Bereich des Minimums im Diagramm bei Auftragung der Kontaktwinkelhysterese gegen die Zeitdauer der Oxidationsbehandlung liegt.

Dies kann dadurch erreicht werden, dass man die Oberfläche des Metallimplantates mit einem Oxidationsmittel behandelt, indem man das bevorzugt entfettete Metallimplantat in heißer Chromschwefelsäure - bevorzugt hat die Chromschwefelsäure dabei eine Dichte von mehr als 1,40 g/cm³ - mit einer Temperatur von oberhalb von 200°C schockerhitzt, d.h. durch Eintauchen innerhalb von wenigen Sekunden auf die Temperatur der Chromschwefelsäure erhitzt, und dort bei dieser Temperatur für einen Zeitraum von 10 bis zu 90 Minuten, bevorzugt bis zu 60 Minuten, besonders bis zu 30 Minuten belässt und danach direkt nach der Entnahme das Metallimplantat innerhalb eines Zeitraumes von unter einer Minute, bevorzugt innerhalb von wenigen Sekunden auf Raumtemperatur abkühlt. Dies kann vorzugsweise dadurch erfolgen, dass man das Metallimplantat in konzentrierter Schwefelsäure mit einer Temperatur von 15°C bis 25°C durch Eintauchen abschreckt. Um Reste von Säure und, falls vorhanden, implantatfremde Metallionen, z.B. Chromionen, zu entfernen, wird die Oberfläche des Metallimplantates in mehreren Waschschritten (bis zu 15) mit destilliertem Wasser gewaschen. Falls auf der Oberfläche des Metallimplantates danach noch Chromionen nachzuweisen sind, kann das Metallimplantat mit einer Lösung eines Komplexierungsmittels solange behandelt werden, bis keine Metallionen mehr nachweisbar sind. Seitens der Erfinder wurde überraschenderweise herausgefunden, dass sich bei Verwendung von EDTA als Komplexierungsmittel die Lösung braunviolett violett verfärbt, wenn Chrom aus den Proben herausgelöst wird. Entsprechend schlagen die Erfinder für den Fall vor, dass die Proben so lange in 10% EDTA (1-3 x) bei pH 7, falls erforderlich auch in siedender EDTA-Lösung, gewaschen werden, bis keine Verfärbung durch Chromionen mehr auftritt.

Mittels dieses Verfahrens ist somit ein Implantat mit einer ultrahydrophilen Oberfläche erhältlich, das gemäß dem erfindungsgemäßen Verfahren lagerfähig gemacht werden kann. Die Erfindung ist gerichtet auf ein Verfahren nach Anspruch 1.

Hierzu haben die Erfinder Versuche durchgeführt, die im Vergleich zu den im Stand der Technik bekannten Lehren überraschende Ergebnisse ergaben. Aufgrund der Aufwendigkeit von Nassverpackungen zur Konservierung hydrophiler Oberflächen auf Implantaten, die bei den ultrahydrophilen Metallimplantaten überraschenderweise auch bei höheren Salzkonzentrationen von mehr als 0,5 M/I lagerstabile Implantate ohne Benetzbarkeitsverlust ermöglichen, wurde auch nach flüssigkeitsfreien Verpackungsmethoden gesucht. Dabei wurde überraschend gefunden, daß ultrahydrophile Titanoberflächen, auf denen man Salzlösungen evaporieren ließ, stabil gegen den Benetzbarkeitsverlust wurden. Zu diesen Salzlösungen gehören beispielsweise 1 M Neutralsalzlösungen wie 1M NaCl, oder auch 5 mM EDTA pH 7,0. Als besonders gut geeignete Lösung erwies sich die Puffermischung mit Gehalten:
120 bis 150 mM NaCl
7,5 bis 8,5 mM Na₂HPO₄
2,5 bis 3,5 mM KCl
1,0 bis 2,0 mM KH₂PO₄
bei einem pH-Wert von 7,2 bis 7,6.

Die Evaporierung kann unter Schutzgas oder in atmosphärischer Luft erfolgen, wobei letztere wegen der Einfachheit standardmäßige Verwendung gefunden hat.

Auf der so behandelten Metalloberfläche bildete sich nach Evaporation eine feine makroskopisch unsichtbare "Exsikkationsschicht", die erfindungsgemäß die Ultrahydrophilizität stabilisiert und schützt. Allgemein lassen sich erfindungemäß Neutralsalzlösungen in Lösung eines einzelnen Salzes oder auch verschiedener Salze in einer Konzentration und Menge verwenden, die gegenüber der ultrahydrohilen Oberfläche inert ist und ausreichend ist, nach dem Abdampfen die Oberfläche des Implantates mit der Exsikkationsschicht zu bedecken. Die Evaporation kann durchgeführt werden, wenn sich das Implantat in der Lösung aus Neutralsalz befindet, oder dann, wenn das Implantat aus der Lösung entnommen wurde und so nur mit einer dünnen Schicht dieser Lösung bedeckt ist.

Noch überraschender war, daß die stabilisierende Wirkung der Exsikkationsschicht nicht durch eine Gammabestrahlung oder andere ionisierende Strahlung aufgehoben wird.

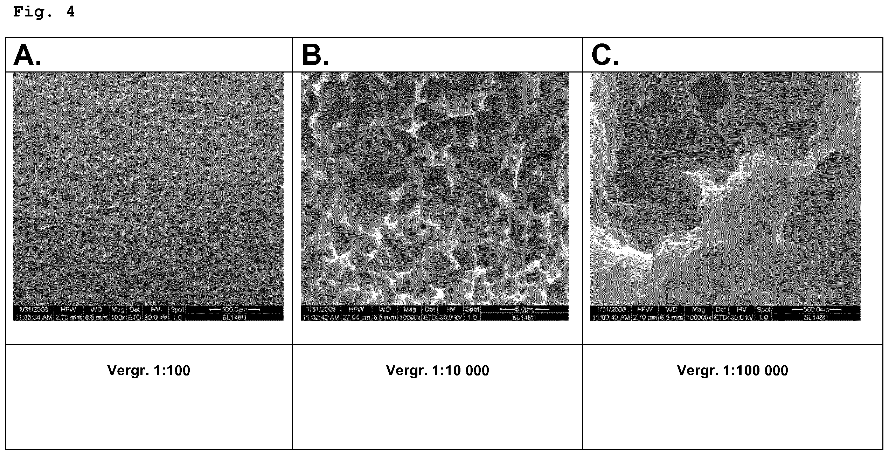

Mit Hilfe der Rasterelektronenmikroskopie und der EDX-Analyse haben die Erfinder auf einfache Weise nachgewiesen, ob eine solche Exsikkationsschicht auf einer Metalloberfläche erfindungsgemäß gebildet wurde (siehe Fig. 4). Ferner zeigte sich, daß in der Regel eine solche Exsikkationsschicht nur dann entsteht, wenn nicht-flüchtige Bestandteile in der verwendeten Lösung vorhanden sind. Eine solche Schicht entsteht beispielsweise nicht, wenn Methanol-, Ethanol- oder Acetonlösungen in Gegenwart ultrahydrophiler Oberflächen evaporiert werden. Der exakte Mechanismus der Stabilisierung der ultrahydrophilen Schicht durch die Exsikkationsschicht ist noch unklar, könnte aber mit elektrostatischen Wechselwirkungen zwischen den Elektroylten in der Pufferlösung und geladenen Gruppen auf der Metalloberfläche zusammenhängen. Da für den Exsikkationsvorgang nur eine sehr dünne Flüssigkeitsschicht von 0,1-0,2 µl/mm² auf der Metalloberfläche nach Herausnahme aus der Pufferlösung für die Evaporation vorhanden ist, ist der Anteil nicht flüchtiger Substanzen in der Exsikkationsschicht nur sehr gering und muß nicht von dem Implantat für die Implantation entfernt werden. Wie oben erwähnt unterscheidet sich die "ultrahydrophile" Oberfläche von der "hydrophilen" dadurch, daß erstere durch Methanol, Ethanol und Aceton stabilisiert werden kann. Hingegen kann sie nicht durch eine 0,15 M NaCl Lösung dauerhaft stabilisiert werden, sondern erst durch eine viel höhere Konzentration von 0,5 M oder höher. Ferner wird der "hydrophile" Charakter der Oberflächen durch Kontaktwinkel zwischen 20-50° charakterisiert, wohingegen "ultrahydrophile" Oberflächen einen Kontaktwinkel von 0-10° aufweisen. Daraus lässt sich ableiten, daß bisher bekannte "hydrophile" Oberflächen von den "ultrahydrophilen" unterschieden werden müssen. Ultrahydrophile Oberflächen lassen sich erfindungsgemäß durch eine Exsikkationsschicht stabilisieren, wobei durchaus denkbar ist, daß die hydrophilen Oberflächen durch die hier beschriebene Methode der Exsikkation von Lösungen mit nicht-flüchtigen Bestandteilen auch zur Stabilisierung einer solchen hydrophilen Oberfläche führen können.

Gemäß diesem Verfahren zur Herstellung eines lagerfähigen Implantates mit einer ultrahydrophilen Oberfläche gibt man das Implantat in eine salzhaltige Lösung, die gegenüber der ultrahydrophilen Oberfläche inert ist und die das Implantat allseits umschließt. Hierbei kann auch ein auf eine andere Weise hergestelltes Implantat eingesetzt werden, das ähnliche Eigenschaften der Hydrophilizität mit der o.a. Kontaktwinkelhysterese von weniger als 5° bei Benetzung mit Wasser besitzt. Wie oben erwähnt, kann die Salzlösung eine Lösung eines einzelnen Salzes oder eine Kombination von verschiedenen Salzen in Wasser sein, wobei das Salz ausgewählt wird aus der Gruppe, die besteht aus der Gruppe der Salze mit einem Anion aus SO₄⁻⁻, HPO₄⁻⁻ , CH₃COO⁻, Cl, Br-, NO₄⁻, ClO₄⁻, I⁻, CNS⁻, ClCH₂COO⁻, F₃CCOO⁻, Cl₂CHCOO⁻, Cl₃CCOO⁻, Br₃CCOO⁻ oder einem Kation aus NH₄⁺, Rb⁺, K⁺, Na⁺, Cs⁺, Li⁺, Mg⁺⁺, Ca⁺⁺, Ba⁺⁺ sowie (CH₃)₄N⁺, (C₂H₅)₄N⁺,(C₃H₇)₄N⁺, (C₄H₉)₄N⁺.

Eine Weiterbildung des erfindungsgemäßen Verfahren umfasst dann den zusätzlichen Schritt, dass man das Implantat in der salzhaltigen Lösung, die eine Gesamtionenkonzentration von mehr als 0,5 Mol/I, vorzugsweise mehr als 1 Mol/I, hat, in eine Transportverpackung einbringt und die Transportverpackung gas- und flüssigkeitsdicht verschließt.

Auf diese Weise wird erfindungsgemäß auch eine Feuchtverpackung bereitgestellt, die das Implantat mit einer ultrahydrophilen Oberfläche zuverlässig vor einem Abbau der Ultrahydrophilie schützt.

Obgleich so eine Langzeitlagerung bereits ermöglicht wird, ist es aus Gründen der einfacheren Handhabung bevorzugt, dass man das Implantat in einer salzhaltigen Lösung, ohne dass man es direkt in eine Verpackung einbringt, so temperaturschonend, ohne die Implantateigenschaften nachteilig zu beeinflussen, einem Verfahren unterzieht, bei dem man die salzhaltige Lösung zur Trockene eindampft. Hierzu ist es ausreichend, dass die Salzlösung eine niedrigere Konzentration als für die Nassverpackung aufweist, da die Konzentration im Verlauf des "Eindampfen" steigt und bei Bildung der Exsikkationsschicht die Löslichkeitsgrente des Salzes übersteigt.

Dabei verwendet man die salzhaltige Lösung vorzugsweise in einer Menge und mit einer Salzkonzentration, die nach dem Eindampfen eine zumindest die ultrahydrophile Oberfläche des Implantates bedeckende Salzschicht mit einer Schichtdicke von vorzugsweise 1 bis 500 µm ergibt. Besonders bevorzugt ergibt die salzhaltige Lösung nach dem Eindampfen eine das Implantat allseits umschließende Salzschicht.

Das so erhaltene Implantat weist somit die von den Erfindern so genannte Exsikkationsschicht auf, die die ultrahydrophile Oberfläche des Implantates bedeckt und schützt. So kann das mit dieser Exsikkationsschicht versehene Implantat in eine Trockenverpackung gegeben und langzeitig gelagert werden. Der Lagerung vorausgehend wird in der Regel eine Sterilisierung des Implantates durchgeführt, wobei die Sterilisierung des Implantates vorzugsweise eine Sterilisierung mit elektromagnetischer Strahlung umfasst.

Demzufolge ist die Erfindung auch auf ein solches lagerfähiges Implantat gerichtet, das nach den verschiedenen Ausführungsformen des erfindungsgemäßen Verfahrens erhältlich ist.

Die Erfindung wird auch anhand der beigefügten Figuren weiter erläutert. Dabei zeigen:
Fig. 1 ein Diagramm zur Größe des Vor- bzw. Rückzugswinkels gegen die Behandlungsdauer einer Metalloberfläche mit Chromschwefelsäure;
Fig. 2 elektronenmikroskopische Abbildungen einer ultrahydrophilen, nanostrukturierten Oberfläche auf cp-Titan durch das Chromschwefelsäureverfahren in verschiedenen Vergrößerungen;
Fig. 3 ein Diagramm zur Freisetzungsrate von BMP von verschieden behandelten Metalloberflächen;
Fig. 4 rasterelektronenmikroskopische Aufnahmen von chromschwefelsäurebehandelten SLA-Titanplättchen (14 x 14 x 1.5 mm) nach Gammasterilisierung in Exsikkationspuffer ( 60 min CSS mit HNOs, mit Abschrecken, gammasterilisiert in PBS, θ = 0°); und
Fig. 5 die EDX-Analyse eines ultrahydrophilen Plättchens mit Exsikkationsschicht nach Gammasteriliserung (A) und nach Entfernung der Exsikkationsschicht mit Wasser (B).

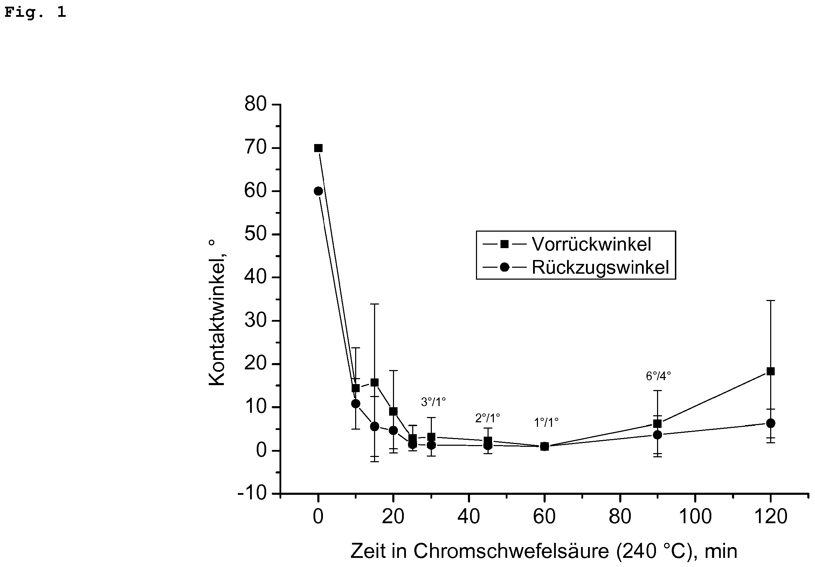

Wie **Fig.** 1 zeigt, führt die Behandlung der Metalloberfläche mit Chromschwefelsäure zur Herstellung ultrahydrophiler Metalloberflächen zu überraschenden Ergebnissen. Dazu wurden Titanplättchen bei 240 °C in konzentrierter Chromschwefelsäure inkubiert. Wie überraschenderweise festgestellt, werden ultrahydrophile Oberflächen (Kontaktwinkel: < 10°; Kontaktwinkelhysterese: ~ 0°; angegeben sind Standardabweichungen (n = 5), in einem Zeitfenster von 30-60 Minuten erhalten.

Ähnliche Minimum-Kurven haben die Erfinder bei 316L Stahl, Titanlegierungen und Cobalt-Chrom Legierungen gefunden. In **Tabelle 1** ist dargestellt, daß man auf der ultrahydrophilen Oberfläche vier- bis fünfmal mehr BMP-2 binden (adsorbieren) kann als auf der Kontrolle. Die gebundene Menge BMP-2 auf der unbehandelten Titanoberfläche liegt noch unter der mit HNO₃ behandelten Oberfläche.

Diese ultrahydrophilen Oberflächen besitzen eine sehr hohe Oberflächenenergie, die im ultrahydrophilen Bereich (Kontaktwinkel < 11°; Kontaktwinkelhysterese ~ 0°) eine kritische Oberflächenspannung yc = 710-720 µN/cm (71-72 dyne/cm) aufweist. Die hohe Oberflächenenergie führt zur Adsorption geeigneter Proteine.

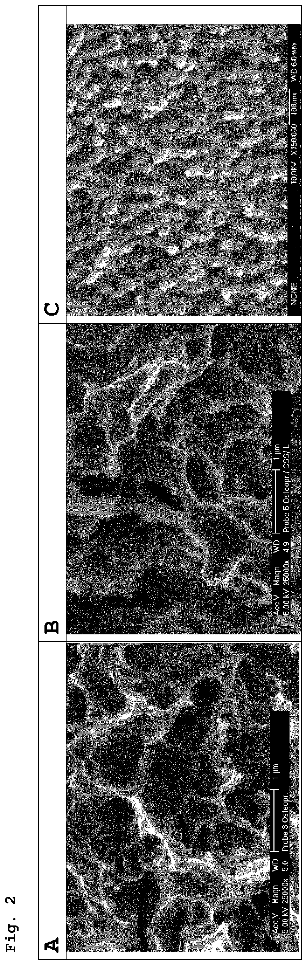

Bei der Ausbildung der ultrahydrophilen Oberfläche wird eine neuartige Nanostruktur durch die neue Chromschwefelsäurebehandlung erzeugt (Fig. 2). Auf der vor der Behandlung glatten Titanoberfläche werden globuläre, untereinander zusammenhängende Gefüge in einem Durchmesser von 50-100 nm ausgebildet, zwischen denen Nanoporen in einem Durchmesser von 50-100 nm vorhanden sind. Diese Nanostrukturen sind vermutlich an der Ausbildung der Ultrahydrophilizität beteiligt. Die Bedingungen sind hierzu im Einzelnen für die Abbildungen A, B und C:

### A. Gereinigte Industriestandard SLA-Oberfläche (Sand-blasted, Large-grit, Acidetched) in 25.000-facher Vergrößerung.

Die Oberfläche wurde mit Korund sandgestrahlt und dann in einem Säurebad (HCl/H₂SO₄) geätzt. Die Oberfläche zeigt eine glatte Mikrostruktur ohne Anzeichen einer Nanostruktur.

### B. SLA-Oberfläche nach Behandlung in Chromschwefelsäure bei 240 °C für 60 Minuten in 25.000-facher Vergrößerung.

Durch die Chromschwefelsäure ist funktionell eine ultrahydrophile Oberfläche und strukturell neben der SLA-Mikrostruktur eine "globuläre" Nanostruktur entstanden. Der Durchmesser der untereinander verbundenen Nano-Kugeln liegt bei etwa 50-100 nm, wobei sich Nanoporen in der gleichen Größenordnung gebildet haben.

### C. Darstellung der globulären Nanostruktur bei 150.000-facher Vergrößerung auf einer elektropolierten Titanoberfläche.

Die Nanokügelchen besitzen einen Durchmesser von ca. 50 nm und sind untereinander verbunden. Zwischen den Kügelchen bilden sich Poren in einem Durchmesser von 10-100 nm.

Auf diesen Nanostrukturen können in einem weiteren Schritt Peptide wie Knochenwachstumsfaktoren mittels physisorptiver oder chemisorptiver Bindung, vermutlich aufgrund hydrophiler Wechselwirkungen auf dem Implantatmaterial immobilisiert werden. Dadurch wird ermöglicht, eine chemotaktisch wirkende und/oder biologisch aktive, eine sogenannte juxtakrine, Implantatoberfläche auszubilden, die zur Ansiedlung, Proliferation und Ausdifferenzierung von Knochenzellen führt. So lassen sich sogenannte aktive Implantate bereitstellen, die bei von der Oberfläche freigesetzten Molekülen auch auf eine Entfernung von 500 bis 1000 µm eine chemotaktische Wirkung auf Zellen, im Falle von BMPs auf Osteoblasten, zeigen.

Bevorzugt wird die ausreichende Beladung der oxidierten Metalloberfläche dadurch erzielt, dass man die Peptide in einer physiologischen Pufferlösung in einer Konzentration, die ausreicht, eine Beladung von mehr als 200 ng/cm², bevorzugt mehr als 500 ng/cm², und mehr bevorzugt von mehr als 1000 ng/cm² des Peptids auf der Oxidoberfläche des Metallimplantates zu erzielen, aufbringt.

In der Regel wird diese Beladung mit einer physiologischen Pufferlösung von Peptiden in einer Konzentration von mehr als 1 µg/ml, bevorzugt mehr als 200 µg/ml Pufferlösung erzielt.

Verfahrensgemäß sind die Peptide Biomoleküle, die vorteilhaft für die Biokompatibilität des Implantates sind, indem sie einer möglichen Abstoßung des Implantates entgegenwirken und/oder das Einwachsen des Implantates fördern.

Wie oben erwähnt, können als Peptide bevorzugt Proteine aus der Klasse der TGF-Proteine, insbesondere das Knochenwachstum fördernde Proteine aus der Klasse der Knochenwachstumsfaktoren "Bone Morphogenic Proteins", oder der Klasse der Gefäßwachstumsfaktoren wie VEGF oder Angiotropin oder auch Ubiquitin verwendet werden. Unter der Bezeichnung "Transforming Growth Factor" (TGF) sind insbesondere die Gruppe (Subgruppe) der (i) "Transforming Growth Factors beta" (TGF-ß) sowie die Gruppe (Subgruppe) der (ii) Bone Morphogenetic Proteine (BMP) zu verstehen. Letztere sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von alkalischer Phosphatase, Hormonrezeptoren, knochenspezifischer Substanzen wie Kollagen Typ 1, Osteocalcin, Osteopontin, Osteonectin, Bone Sialoprotein (BSP) und schließlich die Mineralisation.

Vorteilhaft kann zur Immobilisierung ein Protein dieser Klasse allein, in Kombination mit weiteren Mitgliedern dieser Klasse oder auch zusammen mit Biomolekülen wie Proteinen anderer Klassen oder niedermolekularen Hormonen oder auch Antibiotika zur Verbesserung der Immunabwehr eingesetzt werden. Dabei können diese weiteren Moleküle auch über im physiologischen Milieu spaltbare Bindungen auf der Oberfläche immobilisiert werden.

Seitens der Erfinder wurde bereits früher gefunden, dass die Anzahl der Oxidgruppen überraschenderweise dadurch erhöht werden kann, dass die Oberfläche des Metalls mit heißer, vorzugsweise bodensatzfreier, Chromschwefelsäure behandelt wird. Im Gegensatz zu der Erwartung, dass sich das Metall unter diesen Bedingungen auflöst, wird bei Verwendung dieser Säure eine im Wesentlichen gleichmäßige 5-50 nm dicke hydrophile Oxidschicht auf der Oberfläche des Metalls erzeugt. Das Verfahren ist so schonend, daß selbst koronare Gefäßstützen, sogenannte Stents (die z.B. aus Edelstahl oder Titan gefertigt sein können) ohne Zerstörung des dünnen empfindlichen Gitterwerkes (50-150 µm Durchmesser) beschichtet werden können. Insbesondere eignen sich als Materialien zur Oxidbehandlung mit verdünnter Säure gereinigte Übergangsmetalloberflächen wie Titan, Stahl, Stahllegierungen wie Cr-Mo-Stahl oder mit Chromschwefelsäure veredelte Stahl- oder Reintitanoberflächen oder Titanlegierungen.

Sowohl bei polierten als auch bei sandgestrahlten (SLA-Oberflächen) oder mit Metallplasmen (z.B. Titan-Plasma- Spray, TPS) beschichteten Implantaten kann die ultrahydrophile Oxidschicht nach der Behandlung der Metalloberfläche unter definierten Bedingungen eine Dicke von 10 nm bis zu 300 nm besitzen und in Form von Nanostrukturen, wie in Fig. 2 gezeigt, unterschiedlicher Geometrien (beispielsweise rund oder polygonal) aufgebaut sein. Als Metall für das Implantat können dabei Reintitan oder Titanlegierungen (z.B. TiAIV4, TiAIFe2,5), Aluminium oder rostfreier Stahl (z.B. V2A, V4A, Chrom-Nickel 316L, Cr-Mo-Stahl) eingesetzt werden. Eine handelsübliche Chromschwefelsäure mit 92 Gew.-% H₂SO₄ , 1,3 Gew.-% CrO₃ und einer Dichte von 1,8 g/cm³ , wie beispielsweise von der Firma Merck erhältlich, wird bevorzugt zur Erzielung einer dünnen glatten Schicht aus Metalloxid verwendet.

Das neue Verfahren erlaubt, daß im Gegensatz zu früheren Verfahren in allen Fällen ultrahydrophile Oberflächen hergestellt werden können. Die drei bevorzugten Verfahrensschritte: (i) die neue CSS-Behandlung (Schockerhitzen), (ii) das Abschrecken in konz. Schwefelsäure und (iii) die neue EDTA-Waschmethode senken den Chromgehalt (EDX-Methode) auf der Oberfläche unter die Nachweisgrenze ab. Die EDX-Nachweisgrenze liegt bei 0,2-0,5 Atom%. Die neuen chromfreien ultrahvdrophilen Oberflächen zeigen die besonderen weiter unten gezeigten neuen Eigenschaften bezüglich der BMP-2-Bindung und der Stabilisierung durch die Salzschicht.

Wenn eine dickere Metalloxidschicht (> 1000 nm) an der Metalloberfläche und/oder bevorzugt eine Oxidschicht mit kleinen Mikro- und Nanoporen versehen werden soll, wird die oben beschriebene Chromschwefelsäure mit Wasser auf eine Dichte von 1,5 bis 1,6 g/cm³ verdünnt. Bei einer dann folgenden wie oben beschriebenen Behandlung der Metallimplantatoberfläche mit der so verdünnten Säure wird eine "rauhe" Oberflächenschicht mit Vertiefungen und Poren ausgebildet, so dass die zur Beladung mit Peptiden zur Verfügung stehende Oberfläche vergrößert wird. Durch Einstellung unterschiedlicher Dichten der Chromschwefelsäure und unterschiedlicher Behandlungszeiten und - temperaturen ist es daher möglich, eine Vielzahl verschiedener Oxidschichten unterschiedlicher Eigenschaften auf Metalloberflächen mit hoher Haftfestigkeit aufzubringen.

Die durch die Chromschwefelsäure erzeugte ultrahydrophile Oberfläche kann die hydrophilen Eigenschaften bei längerer Lagerung an der Luft und in reinem Wasser verlieren. Der Kontaktwinkel kann unter diesen Bedingungen nach 1-2 Stunden auf Werte von 20-40° steigen. Die ultrahydrophile Oberfläche kann erfindungsgemäß mittels einer Salzlösung als Stabilisierungsmittel stabilisiert werden. Bevorzugt ist, wie oben erwähnt, eine Stabilisierung durch verschiedene wässrige Salzlösungen möglich, die nach ihrem Aussalzeffekt gegenüber Proteinen gereiht werden können (Tabelle 2). Dabei handelt es sich beispielsweise um die Anionen SO₄⁻⁻, HPO₄⁻⁻,CH₃COO⁻, Cl⁻, Br⁻, NO₄⁻, ClO₄⁻, I⁻, CNS⁻, ClCH₂COO⁻, F₃CCOO⁻, Cl₂CHCOO⁻, Cl₃CCOO⁻, Br₃CCOO⁻ oder die Kationen NH₄⁺, Rb⁺, K⁺, Na⁺, Cs⁺, Li⁺, Mg⁺⁺, Ca⁺⁺, Ba⁺⁺ sowie Tetraalkylammoniumkationen wie (CH₃)₄N⁺, (C₂H₅)₄N⁺, (C₃H₇)₄N⁺, (C₄H₉)₄N⁺. Bevorzugt werden NaCI-Salzlösungen über 0,15 Mol/I, besonders bevorzugt über 0,5 Mol/I ganz besonders bevorzugt im Bereich von 1 Mol/I. In solchen Lösungen sind die ultrahydrophilen Oberflächen nahezu unbegrenzt stabil. Solche hohen Salzkonzentrationen entstehen für kurze Zeiten während der Evaporierung auch aus verdünnten Puffermischungen wie oben angegeben. Bevorzugt ist eine Salzkonzentration in der Pufferlösung von 135 bis 140 mM NaCl, 8 bis 8,2 mM Na₂HPO₄, 2,6 bis 2,8 mM KCl, 1,4 bis 1,6 mM KH₂PO₄ bei einem pH im Bereich von 7,3 bis 7,5. Die Evaporierung führt bis zur Trockene zu hohen lokalen Salzkonzentrationen. Dabei kann das HPO₄⁻⁻, welches wesentlich stärkere aussalzende Eigenschaften als das Cl⁻ besitzt, einen besonderen stabilisierenden Einfluß auf die Oxidschicht ausüben.

Die Erfindung ist daher auf ein Verfahren gerichtet, die mit einer Oxidschicht mit Nanostrukturen versehenen Implantate mit Hilfe derartiger "Stabilisierungsmittel" lagerstabil zu machen.

Die erfindungsgemäß beschichteten Implantate sind langzeit-lagerstabil und können nach dem Abwaschen der Beschichtung aus Salzen oder organischem Beschichtungsmittel zur Beladung mit den als Mediatoren wirkenden Peptiden eingesetzt werden.

Dies ist ein Verfahren zur Beladung der Oberfläche eines Implantates mit Peptiden, bei dem man auf die Oberfläche des Implantates Peptide aufgibt, die infolge physisorptiver oder chemisorptiver Wechselwirkungen zwischen den Peptiden und der ultrahydrophilen Oberfläche des Implantates darauf immobilisiert werden.

Dabei verwendet man die Peptide in einer physiologischen Pufferlösung bei einer Konzentration, die ausreicht, eine Beladung von mehr als 200 ng/cm², bevorzugt mehr als 500 ng/cm², und mehr bevorzugt von mehr als 1000 ng/cm² des Peptids auf der Oxidoberfläche des Metallimplantates zu erzielen.

In der Regel werden die Peptide in einer physiologischen Pufferlösung so in einer Konzentration von mehr als 1 µg/ml, bevorzugt mehr als 200 µg/ml Pufferlösung verwendet.

Verfahrensgemäß werden als Peptide Wachstumsfaktoren aus der Klasse der TGF-Proteine, insbesondere der BMP-Proteine, bevorzugt BMP-2 oder BMP-7, der Gefäßwachstumsfaktoren wie VEGF oder Angiotropin, Ubiquitin, Antibiotika oder Mischungen davon verwendet.

Falls unter den Kopplungsbedingungen die eingesetzten Mediatoren im Medium schwerlöslich sind, kann die Löslichkeit durch Zugabe von Tensiden/Detergentien erhöht und die Reaktion durchgeführt werden. So können bei pH-Werten > 6 schwerlösliche Knochenwachstumsfaktoren und andere Mediatoren durch ionische oder nichtionische Detergentien im Konzentrationsbereich 0,05-10%, vorzugsweise 1 -5 Gew.%, insbesondere bei 0,066% SDS bei pH-Werten > 6, insbesondere bei pH 8-12, ganz besonders pH 9-11, speziell pH 10,0 für Bindungsverfahren im alkalischen pH-Bereich ohne Verlust der biologischen Aktivität in Lösung gehalten werden. Somit ist dies ein Verfahren zur Herstellung von mit Peptiden beschichteten Implantaten, bei dem das Implantat mit einer ultrahydrophilen Oberfläche mit einer bevorzugt alkalischen Pufferlösung, die eine oder mehrere Detergentien enthält, behandelt wird. Ein solches Verfahren kann besonders eine Behandlung mit einem NBS-Puffer aus 125 mM NaBorat Puffer, 0,066% Natriumdodecylsulfat (pH 10,0) umfassen.

Entsprechend ist dies ein Verfahren zur Beladung von Implantaten mit Knochenwachstumsfaktoren, insbesondere BMP-2, bei dem die ultrahydrohile Oberfläche des Implantates mit einer Lösung des Knochenwachstumsfaktors bei einem pH-Wert von 9 bis 11, bevorzugt 10, behandelt wird. Hierzu kann beispielsweise ein Implantat, beispielsweise das aus der Trockenverpackung entnommene Implantat, das noch mit der Exsikkationsschicht bedeckt ist, vorzugsweise direkt mit einer gepufferten Lösung des Knochenwachstumsfaktors bei einem pH-Wert von 9 bis 11, bevorzugt 10, behandelt werden, ohne dass die Exsikkationsschicht zuvor abgewaschen werden muss.

Somit ist es möglich, eine Beschichtung der ultrahydrohilen Oberfläche eines Implantates, das aus metallischen Materialien wie Reintitan, metallischen Titanlegierungen, Chrom/Nickel/Aluminium/Vanadium/Kobalt-Legierungen (z.B. TiAIV4, TiAIFe2,5), Edelstählen (z.B. V2A, V4A, Chrom-Nickel 316L), keramischen Materialien insbesondere Hydroxylapatit, Aluminiumoxid, oder aus Kombinationen der metallischen Materialien mit keramischen Materialien davon, bei der das metallische Material als Verbundmaterial mit keramischem Material vorliegt, ausgewählt wird, mit Knochenwachstumsfaktoren, insbesondere BMP-2 zu erzielen, wobei die Beschichtung der ultrahydrophilen Oberfläche in wässriger gepufferter Lösung entweder im sauren Bereich im Bereich zwischen pH 4 und 5, insbesondere bei pH 4,5, oder im schwach alkalischen Bereich zwischen pH 9 und 11, bevorzugt pH 10 durchgeführt wird. Die Beschichtung im alkalischen Bereich kann vorteilhaft in Gegenwart von Detergentien wie SDS durchgeführt werden.

Eine besonders bevorzugte Ausführungsform dieses Verfahrens ist derart ausgebildet, dass man auf die ultrahydrophile Oberfläche des Implantates BMP-2 oder BMP-7 in einer physiologischen Pufferlösung in einer Konzentration von mehr als 1 µg BMP-2 oder BMP-7/ml Pufferlösung, bevorzugt mehr als 200 µg BMP-2 oder BMP-7/ml Pufferlösung aufbringt. Diese vorgenannten Konzentrationen reichen in der Regel aus, eine Beladung von mehr als 200 ng BMP-2 oder BMP-7/cm², bevorzugt mehr als 500 ng BMP-2 oder BMP-7/cm², und mehr bevorzugt von mehr als 1000 ng BMP-2 oder BMP-7/cm² des Peptids auf der oxidierten Oberfläche des Metallimplantates zu erzielen.

Die so mittels dieses Verfahrens hergestellten Implantate mit einer ultrahydrophilen Oberfläche sind Implantate, bei denen das Implantatmaterial aus Titan, Titanlegierungen, Aluminium, rostfreiem Stahl, Stahllegierungen, chromhaltigen Legierungen, keramischen Materialien wie Hydroxapatit oder Kombinationen davon besteht. Das Implantat kann dabei eine Gelenk- oder Knochenprothese, ein Zahnimplantat oder insbesondere eine mit einem Peptid, z.B. BMP-2, beschichtete koronare Gefäßstütze (ein sogenannter koronarer Stent, Länge ca. 10 mm) sein, um die Spätkomplikation Restenose, die durch eine Proliferation von glatten Gefäßmuskelzellen hervorgerufen wird, therapeutisch zu verhindern oder zu mildern, um damit die Einheilung und Verträglichkeit zu fördern.

Der Einfluss der nach dem Verfahren modifizierten Materialien auf Knochenzellen wurde im Tierversuch untersucht, wobei die modifizierten Materialien zu diesem Zweck in Plättchen- oder Hantelform hergestellt wurden. Dabei wurde beobachtet, dass es 4 Wochen nach dem Einbringen in die Tiere zu einer beschleunigten Knochenbildung mit Kontakt zur Implantatoberfläche durch BMP-2 auf den Materialien kam.

Anhand der folgenden Beispiele wird dies weiter veranschaulicht.

### Modifikation von Metallen (Titan, 316 L rostfreier Stahl):

In den im Folgenden beschriebenen Versuchen wurden entweder mechanisch polierte/elektropolierte, anodisch oxidierte Titanplättchen, mit anderen Säuren vorgeätzte, sandgestrahlte oder mit poröser Titanlegierung plasmagespritzte Titanlegierungsplättchen mit oder ohne Chromschwefelsäureveredelung eingesetzt. Gleichermaßen werden rostfreie mechanisch polierte/elektropolierte Stähle mit oder ohne Chromschwefelsäureveredelung eingesetzt.

### Reinigungsverfahren

Vor jedem Einsatz wurden die Metalle gereinigt durch Erhitzen auf 80 °C in 5% HNO₃ für 2 Stunden. Nach erneutem Waschen in Wasser wurden die Plättchen durch Waschen in 30 ml trockenem Methanol getrocknet. Danach wurden sie entweder direkt weiterverwendet oder mit Chromschwefelsäure veredelt.

### Chromschwefelsäureveredelung

Bei der Chromschwefelsäureveredelung wurden die Titanplättchen bei 210-240 °C in Chromschwefelsäure (92% H₂SO₄, 1.35 CrO₃) schockerhitzt, für 30-90 min bei dieser Temperatur inkubiert und dann mit conc. Schwefelsäure bei Raumtemperatur abgeschreckt. Danach wurden die Metallproben mit 10 x 50 ml Wasser gewaschen, mit 2 x 30 min 10 % EDTA ((pH 7) Ultraschall) und anschließend 1-3 x 30 min in siedenden 10 % EDTA (pH 7) behandelt und danach für 30 min mit siedendes Wasser gewaschen und mit Wasser gespült. Auf diese Weise kann eine ultrahydrophile Oberfläche bereitgestellt werden, die im Wesentlichen chromatfrei ist, d.h. es lassen sich keine freien Chromionen auf der Oberfläche mehr nachweisen.

### Beispiel 1 - Immobilisierung von rhBMP-2 auf ultrahydrophilen Titanplättchen

Die vorbehandelten Titanplättchen wurden mit 125 mM NaBorat Puffer, 0,066% Natriumdodecylsulfat, pH 10,0 gewaschen und äquilibriert. BMP-2, das zunächst in 50 mM Tris, pH 8.0. 1000 mM NaCl, 5 mM EDTA, 33 mM 3-[(3-Cholamidopropyl)dimethylammonio]-propansulfonsäure Puffer (= CPDP-Puffer) vorlag, wurde gegen 125 mM NaBorat Puffer, 0,066% Natriumdodecylsulfat, pH 10,0 (= NBS-Puffer) dialysiert und in einer Konzentration von 0,2-0,3 mg/ml 12-14 Stunden bei Raumtemperatur unter Schütteln mit den Titanplättchen inkubiert. Danach wurden sie 4 x mit Boratpuffer und anschließend mit Wasser gewaschen.

**Tabelle 1 - Immobilisierung von rhBMP-2 auf HNO₃-behandelten oder mit Chromschwefelsäure behandelten Titanplättchen (5 x 10 x 1 mm)**

| Modifizierung von elektropoliertem Titan | Immobilisiertes 125I-rhBMP-2 [ng/cm2] |
|---|---|
| HNO3-behandelt (θᵥₒᵣ/θ_{rück} = 40°/20°) | 273 ± 107 (4) |
| ultrahydrophil (θᵥₒᵣ/θ_{rück} = 1°/1°) | 1272 ± 636 (4) |

| | |
|---|---|
| Immobilisierungspuffer: 125 mM Borat/0,066 % SDS, pH 10,0, C_{rhBMP-2} = 0,25 mg/ml, n = 4. θᵥₒᵣ: dynamischer Vorrückwinkel, θ_{rück}: dynamischer Rückzugswinkel | |

**Tabelle 2 - Reihung der Salze nach ihrem Aussalz- bzw. Einsalzeffekt gegenüber Proteinen.**

| Aussalzeffekt | Einsalzeffekt |
|---|---|
| Anionisch: | |
| SO₄⁻⁻ > HPO₄⁻⁻ > CH₃COO⁻ > Cl⁻ > Br⁻ > NO₄⁻ > ClO₄⁻ > I⁻ > CNS⁻ CH₃COO⁻ > ClCH₂COO⁻ > F₃CCOO⁻ > Cl₂CHCOO⁻ > Cl₃CCOO⁻ > Br₃CCOO⁻ | |
| Kationisch: | |
| (CH₃)₄N⁺ > NH₄⁺ > Rb⁺, K⁺, Na⁺, Cs⁺ > Li⁺ > Mg⁺⁺ > Ca⁺⁺ > Ba⁺⁺ | |
| Kationisch/ hydrophob | |
| (CH₃)₄N⁺ > (C₂H₅)₄N⁺ >> (C₃H₇)₄N⁺, (C₄H₉)₄N⁺ | |

Die Wirkung der Salze erfolgt u.a. über die Wasserstruktur. Die Salze stabilisieren bzw. destabilisieren die ultrahydrophile Oberfläche über die an der Titanoberfläche gebundenen Wassermoleküle und ionischen Gruppen. Bevorzugt werden NaCl Salzlösungen über 0,15 Mol/I, besonders bevorzugt über 0,5 M/I, ganz besonders bevorzugt im Bereich von 1 Mol/I.

### Beispiel 2 - Freisetzung von rhBMP-2 von ultrahydrophilen Titanplättchen

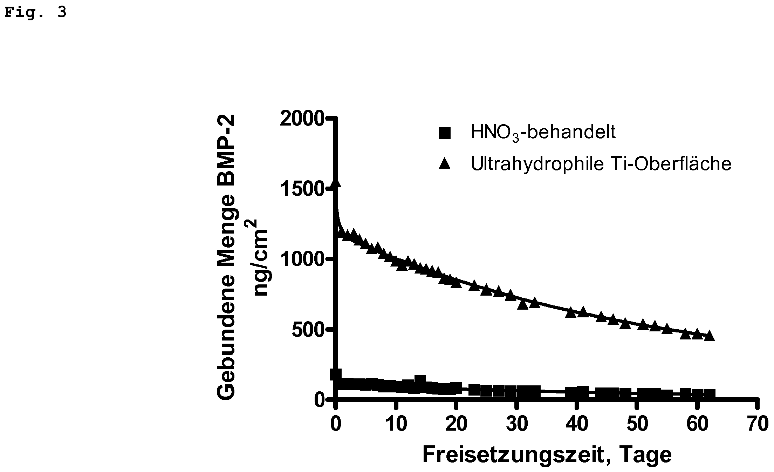

Wie in Fig. 3 gezeigt, ist die Adsorption von rhBMP-2 an einer ultrahydrophilen Titanoberfläche und die Freisetzung von der Oberfläche deutlich gegenüber lediglich mit verdünnter HNO₃ zur Reinigung behandelter Titanoberfläche verbessert, wie anhand der in Fig. 3 dargestellten Freisetzungskinetik von rhBMP-2 von einer ultrahydrophilen Titanoberfläche zu sehen ist. Die großen Kapazitätsunterschiede werden auch deutlich. Die gezeigten Freisetzungskurven lassen sich mit einer 3-phasigen Exponentialfunktion anpassen. Im Falle der Kontrolle sind es eigentlich nur 2 Phasen. Die Freisetzung ist über 62 Tage gemessen worden. Die Halbwertezeiten der Freisetzung und die freigesetzten Mengen an rhBMP-2 sind in **Tabelle** 3 angegeben.

**Tabelle 3 - Freisetzung von rhBMP-2 von ultrahydrophilen Titanplättchen**

| Freisetzungsphase | HNO₃-behandelt θᵥₒᵣ/θ_{rück} = 40°/20°) | | Ultrahydrophile Titanoberfläche | |
|---|---|---|---|---|
| | Γo = 181 ng/cm² | | (θᵥₒᵣ/θ_{rück} = 1°/1°) Γo = 1551 ng/cm² | |
| | T1/2 Tage | Freigesetzte Menge/Tag ng/Tag | T1/2 Tage | Freigesetzte Menge/Tag ng/Tag |
| 1. Phase (2 Tage) | 0,28 | 36 | 0,37 | 193 |
| 2. Phase (42 Tage) | 39 | 0,5 | 33 | 14 |
| 3. Phase (18 Tage) | 39 | 0,5 | 231 | 7 |
| | In 62 Tagen Freigesetzte Gesamtmenge: | 102 | | 1100 |

| | | | | |
|---|---|---|---|---|
| Γo: Immobilisierte rhBMP-2 Menge/cm² zum Zeitpunkt t = 0, | | | | |

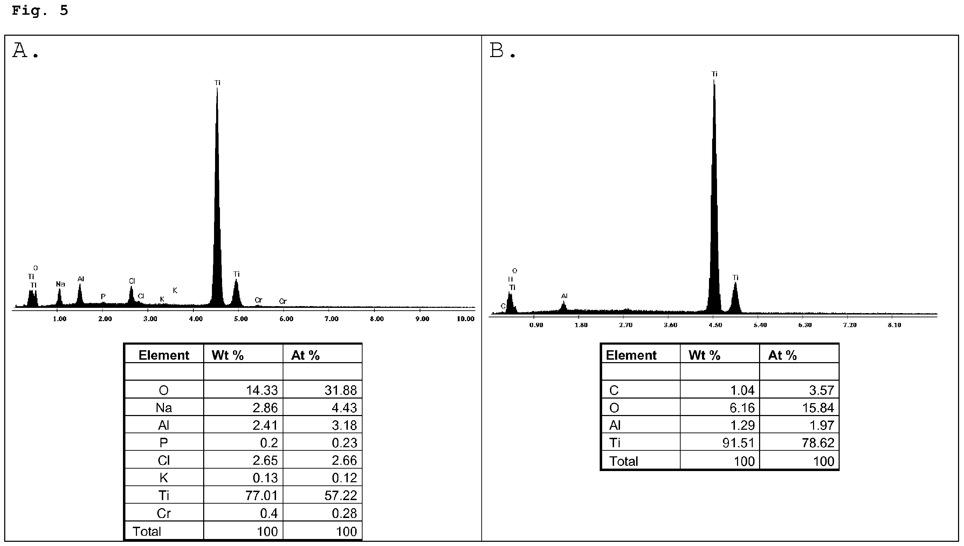

Wie in Figur 4 zu sehen, zeigen die rasterelektronenmikroskopische Aufnahmen von chromschwefelsäurebehandelten SLA-Titanplättchen (14 x 14 x 1.5 mm) nach Gammasterilisierung in Exsikkationspuffer ( 60 min CSS mit HNO₃, mit Abschrecken, gammasterilisiert in PBS, θ = 0°) eine mit Mikrokavernen versehene sterilisierte ultraphile Titanoxidoberfläche mit einer Schutzschicht aus eingetrocknetem Exsikkationspuffer, die nach dem Abwaschen der "Exsikkationsschutzschicht" eine reine ultraphile Titanoxidoberfläche ergibt, wie, wie in Fig. 5 gezeigt, die EDX-Analysen eines ultrahydrophilen Plättchens mit Exsikkationsschicht nach Gammasteriliserung (A) und nach Entfernung der Exsikkationsschicht mit Wasser (B) belegen.

Die Lagerstabilität der ultrahydrophilen Oberflächen wird anhand der Abhängigkeit der dynamischen Kontaktwinkel von mit einer erfindungsgemäßen Exsikkationsschicht versehenen, gammasterilisierten, ultrahydrophilen SLA-Titanplättchen (14 x 14 x 1.5 mm) von der Lagerungszeit gemäß Tabelle 4 gezeigt, wobei man unter SLA-Titanplättchen man Titan-Plättchen versteht, die sandgestrahlte und säuregeätzte Oberflächen besitzen. Wie gezeigt, ist eine "ungeschützte" hydrophile Oberfläche bereits nach wenigen Stunden an der Luft weniger hydrophil, während die Kontaktwinkel der mit der erfindungsgemäßen Exsikkationsschicht versehenen, gammasterilisierten, ultrahydrophilen SLA-Titanplättchen nach bis zu 24 Wochen Lagerung nahezu unverändert bei 0° konstant sind.

**Tabelle 4**

| **Probe** | **Probe vor Chrom-schwefelsäure-behandlung** | | **Lagerungszeit nach Chromschwefelsäurebehandlung (exsikkationsbeschichtet und gammasterilisiert)** | | | | | | | | | | | | | |
|---|---|---|---|---|---|---|---|---|---|---|---|---|---|---|---|---|
| | **18 Std. in Luft Gelagert** | | **Kontrolle (0-Tage)** | | **6 Tage** | | **15 Tage** | | **4 Wochen** | | **8 Wochen** | | **18 Wochen** | | **24 Wochen** | |
| | **Dynamische Kontaktwinkel nach Wilhelmy** | | | | | | | | | | | | | | | |
| | θᵥₒᵣ | θ_{rück} | θᵥₒᵣ | θ_{rück} | θᵥₒᵣ | θ_{rück} | θᵥₒᵣ | θ_{rück} | θᵥₒᵣ | θ_{rück} | θᵥₒᵣ | θ_{rück} | θᵥₒᵣ | θ_{rück} | θᵥₒᵣ | θ_{rück} |
| SLA-1 | 59,2 | 0,0 | 0,0 | 0,0 | 0,0 | 0,0 | 6.1 | 0,0 | 0,0 | 0,0 | 0,0 | 0,0 | 0,0 | 0,0 | 0,0 | 0,0 |
| SLA-2 | 53,3 | 0,0 | 0,0 | 0,0 | 5.7 | 0,0 | 0,0 | 0,0 | 0,0 | 0,0 | 0,0 | 0,0 | 0,0 | 0,0 | 0,0 | 0,0 |
| SLA-3 | 100,8 | 0,0 | 0,0 | 0,0 | 0,0 | 0,0 | 0,0 | 0,0 | 0,0 | 0,0 | 0,0 | 0,0 | 0,0 | 0,0 | 0,0 | 0,0 |
| SLA-4 | 9,3 | 0,0 | 0,0 | 0,0 | 0,0 | 0,0 | 0,0 | 0,0 | 0,0 | 0,0 | 0,0 | 0,0 | 0,0 | 0,0 | 0,0 | 0,0 |
| SLA-5 | 86.5 | 0,0 | 0,0 | 0,0 | 0,0 | 0,0 | 0,0 | 0,0 | 0,0 | 0,0 | 0,0 | 0,0 | 0,0 | 0,0 | 0,0 | 0,0 |
| | | | | | | | | | | | | | | | | |
| Mittelwert (∅) | 78,8 | 0,0 | 0,0 | 0,0 | 1, 1 | 0,0 | 1,2 | 0,0 | 0,0 | 0,0 | 0,0 | 0,0 | 0,0 | 0,0 | 0,0 | 0,0 |
| Standardabw. (s) | 21,3 | 0,0 | 0,0 | 0,0 | 2,5 | 0,0 | 2,7 | 0,0 | 0,0 | 0,0 | 0,0 | 0,0 | 0,0 | 0,0 | 0,0 | 0,0 |

## Patentansprüche

1. Verfahren zur Herstellung eines lagerfähigen Implantates mit einer ultrahydrophilen Oberfläche, bei dem man das Implantat mit einer ultrahydrophilen Oberfläche mit dynamischen Kontaktwinkeln von 0° bis 10° bei Benetzung der Oberfläche des Implantates mit Wasser und mit einer Kontaktwinkelhysterese von weniger als 5° bei Benetzung der Oberfläche des Implantates mit Wasser in eine salzhaltige wässrige Lösung gibt, die gegenüber der Oberfläche inert ist und die das Implantat allseits umschließt, und man die salzhaltige wässrige Lösung unter Bildung einer Exsikkationsschicht, die die Oberfläche des Implantats stabilisiert und schützt, evaporiert, wobei das Implantat aus einem Material, das aus der Gruppe der Metalle, der metallischen Legierungen und Kombinationen davon mit keramischen Materialien ausgewählt wird, besteht und das optional eine Oxidschicht auf der Oberfläche aufweist.

2. Verfahren nach Anspruch 1, wobei die Lösung, in die das Implantat gegeben wird, eine salzhaltige wässrige Lösung ist, die gegenüber der ultrahydrophilen Oberfläche inert ist und die eine Gesamtionenkonzentration von mehr als 0,5 Mol/I, bevorzugt von mehr als 1 Mol/I, hat.

3. Verfahren nach Anspruch 1 oder 2, wobei die salzhaltige wässrige Lösung eine Lösung eines einzelnen Salzes oder eine Kombination von verschiedenen Salzen in Wasser ist, wobei das Salz vorzugsweise ausgewählt ist aus der Gruppe, die besteht aus der Gruppe der Salze mit einem Anion aus SO₄⁻⁻, HPO₄⁻⁻, H₂PO₄⁻, CH₃COO⁻, Cl, Br⁻, NO₄⁻, ClO₄⁻, I⁻, CNS⁻, ClCH₂COO⁻ , F₃CCOO⁻, Cl₂CHCOO⁻, Cl₃CCOO⁻, Br₃CCOO⁻ oder mit einem Kation aus NH₄⁺, Rb⁺, K⁺, Na⁺, Cs⁺, Li⁺, Mg⁺⁺, Ca⁺⁺, Ba⁺⁺ sowie (CH₃)₄N⁺, (C₂H₅)₄N⁺,(C₃H₇)₄N⁺, (C₄H₉)₄N⁺.

4. Verfahren nach einem der Ansprüche 1 bis 3, bei dem man die salzhaltige Lösung in einer Menge und mit einer Salzkonzentration verwendet, die nach dem Evaporieren eine zumindest die ultrahydrophile Oberfläche des Implantates bedeckende Exsikkationsschicht mit einer Schichtdicke von 1 bis 500 µm ergibt.

5. Verfahren nach einem der Ansprüche 1 bis 4, bei dem man eine salzhaltige wässrige Lösung verwendet, die nach dem Evaporieren eine das Implantat allseits umschließende Exsikkationsschicht ergibt.

6. Verfahren nach einem der Ansprüche 1 bis 5, das den zusätzlichen Schritt der Sterilisierung des Implantates umfasst.

7. Verfahren nach Anspruch 6, wobei die Sterilisierung des Implantates eine Sterilisierung mit ionisierender Strahlung, insbesondere elektromagnetischer Strahlung, umfasst.

8. Lagerfähiges Implantat, das nach dem Verfahren nach einem der Ansprüche 1 bis 7 erhältlich ist oder das eine ultrahydrophile Oberfläche, die mit einer Exsikkationsschicht beschichtet ist, aufweist.

9. Lagerfähiges Implantat nach Anspruch 8, wobei das Implantat aus einem Material, das aus der Gruppe der Metalle, der metallischen Legierungen und Kombinationen davon mit keramischen Materialien ausgewählt wird, besteht und das optional eine Oxidschicht auf der Oberfläche aufweist.

10. Lagerfähiges Implantat nach Anspruch 8 bis 9, wobei das eingesetzte Implantatmaterial aus metallischen Materialien wie Reintitan oder metallischen Titanlegierungen, Chrom/Nickel/Aluminium/Vanadium/Kobalt-Legierungen, TiAIV4, TiAIFe2,5, Edelstählen, V2A, V4A, Chrom-Nickel 316L oder aus einer Kombination davon mit keramischen Materialien wie Hydroxyapatit, Aluminiumoxid, bei der das metallische Material aus Verbundmaterial mit keramischen Materialien vorliegt.

11. Lagerfähiges Implantat nach einem der Ansprüche 8 bis 10, wobei die ultrahydrophile Oberfläche eine Kontaktwinkelhysterese von weniger als 5° bei Benetzung der Oberfläche des Implantates mit Wasser aufweist.

12. Lagerfähiges Implantat nach einem der Ansprüche 8 bis 11, wobei die Exsikkationsschicht erzeugt wird, in dem man das Implantat in eine salzhaltige wässrige Lösung, bevorzugt in eine wässrige Neutralsalzlösung, gibt, die gegenüber der ultrahydrophilen Oberfläche inert ist, und die das Implantat allseits umschließt, und man die salzhaltige wässrige Lösung zur Trockene eingedampft.

13. Lagerfähiges Implantat nach Anspruch 12, wobei die Exsikkationsschicht erzeugt wird, in dem man das Implantat in eine salzhaltige wässrige Lösung, bevorzugt in eine wässrige Neutralsalzlösung, gibt, die eine Gesamtionenkonzentration von mehr als 0,5 Mol/I hat.

14. Lagerfähiges Implantat nach einem der Ansprüche 8 bis 13, wobei die Exsikkationsschicht aus einem einzelnen Salz oder aus einer Kombination von verschiedenen Salzen besteht, wobei das Salz vorzugsweise ausgewählt ist aus der Gruppe, die besteht aus der Gruppe der Salze mit einem Anion aus SO₄⁻⁻, HPO₄⁻⁻, H₂PO₄⁻, CH₃COO⁻, Cl, Br-, NO₄⁻, ClO₄⁻, I⁻, CNS⁻, ClCH₂COO⁻ , F₃CCOO⁻, Cl₂CHCOO⁻, Cl₃CCOO⁻, Br₃CCOO⁻ oder mit einem Kation aus NH₄⁺, Rb⁺, K⁺, Na⁺, Cs⁺, Li⁺, Mg⁺⁺, Ca⁺⁺, Ba⁺⁺ sowie (CH₃)₄N⁺, (C₂H₅)₄N⁺,(C₃H₇)₄N⁺, (C₄H₉)₄N⁺.

## Claims

1. A method of producing a storable implant having an ultrahydrophilic surface, comprising placing the implant having an ultrahydrophilic surface with dynamic contact angles of 0° to 10° when the surface of the implant is wetted with water and with a contact angle hysteresis of less than 5° when the surface of the implant is wetted with water, in a salt-containing aqueous solution which is inert with respect to the surface and which surrounds the implant on all sides, and the salt-containing aqueous solution is evaporated to form an exsiccation layer which stabilises and protects the surface of the implant, wherein the implant consists of a material which is selected from the group of metals, metallic alloys and combinations thereof with ceramic materials and which optionally has an oxide layer on the surface.

2. The method of claim 1, wherein the solution into which the implant is placed is a salt-containing aqueous solution which is inert to the ultrahydrophilic surface and which has a total ion concentration of more than 0.5 mol/I, preferably more than 1 mol/I.

3. A process according to claim 1 or 2, wherein the salt-containing aqueous solution is a solution of a single salt or a combination of different salts in water, the salt preferably being selected from the group, consisting of the group of salts with an anion of SO₄ ⁻⁻, HPO₄⁻⁻, H₂ PO₄⁻, CH₃COO⁻, Cl, Br⁻, NO₄⁻ ClO₄⁻ , CNS⁻, ClCH₂ COO⁻, F₃CCOO⁻, Cl₂ CHCOO⁻, Cl₃ CCOO⁻, Br₃ CCOO⁻ or with a cation from NH₄ ⁺, Rb⁺, K⁺, Na⁺, Cs⁺, Li⁺, Mg⁺⁺, Ca⁺⁺, Ba⁺⁺ as well as (CH₃)₄N⁺, (C₂ H5)₄N⁺, (C₃ H7)₄N⁺, (C₄ H9)₄N⁺.

4. The method according to any one of claims 1 to 3, wherein the salt-containing solution is used in an amount and with a salt concentration which, after evaporation, results in an exsiccation layer covering at least the ultrahydrophilic surface of the implant with a layer thickness of 1 to 500 µm.

5. The method according to any one of claims 1 to 4, wherein a salt-containing aqueous solution is used which, after evaporation, produces an exsiccation layer surrounding the implant on all sides.

6. A method according to any one of claims 1 to 5, comprising the additional step of sterilising the implant.

7. The method according to claim 6, wherein the sterilisation of the implant comprises sterilisation with ionising radiation, in particular electromagnetic radiation.

8. A storable implant obtainable by the method of any one of claims 1 to 7, or having an ultrahydrophilic surface coated with a desiccation layer.

9. A storable implant according to claim 8, wherein the implant is made of a material selected from the group consisting of metals, metallic alloys and combinations thereof with ceramic materials, and optionally having an oxide layer on the surface.

10. A storable implant according to claim 8 to 9, wherein the implant material used consists of metallic materials such as pure titanium or metallic titanium alloys, chromium/nickel/aluminium/vanadium/cobalt alloys, TiAIV4, TiAIFe2.5, stainless steels, V2A, V4A, chromium-nickel 316L or a combination thereof with ceramic materials such as hydroxyapatite, alumina, wherein the metallic material is present as a composite material with ceramic materials.

11. A storable implant according to any one of claims 8 to 10, wherein the ultrahydrophilic surface has a contact angle hysteresis of less than 5° when the surface of the implant is wetted with water.

12. A storable implant according to any one of claims 8 to 11, wherein the desiccation layer is produced by placing the implant in a salt-containing aqueous solution, preferably in an aqueous neutral salt solution, which is inert with respect to the ultrahydrophilic surface and which surrounds the implant on all sides, and the salt-containing aqueous solution is evaporated to dryness.

13. A storable implant according to claim 12, wherein the desiccation layer is created by placing the implant in a salt-containing aqueous solution, preferably in an aqueous neutral salt solution, having a total ion concentration of more than 0.5 mol/I.

14. A storable implant according to any one of claims 8 to 13, wherein the desiccation layer consists of a single salt or a combination of different salts, the salt preferably being selected from the group, consisting of the group of salts with an anion of SO4 --, HPO4--, H2 PO4-, CH3 COO-, Cl, Br-, NO4-, ClO4-, CNS-, CICH2 COO-, F₃CCOO⁻, Cl2 CHCOO-, Cl3 CCOO-, Br3 CCOO- or with a cation from NH4 +, Rb+, K+, Na+, Cs+, Li+, Mg++, Ca++, Ba++ as well as (CH3)4N+, (C2 H5)4N+, (C3 H7)4N+, (C4 H9)4N+.

## Revendications

1. Procédé de fabrication d'un implant apte au stockage ayant une surface ultrahydrophile, consistant introduire ledit implant ayant une surface ultrahydrophile, dont les angles de contact dynamiques sont compris entre 0° et 10° lorsque la surface de l'implant est mouillée avec de l'eau et dont l'hystérésis d'angle de contact est inférieure à 5° lorsque la surface de l'implant est mouillé avec de l'eau, dans une solution aqueuse contenant du sel laquelle est inerte vis-à-vis de ladite surface et laquelle vient entourer l'implant de tous les côtés, et à évaporer la solution aqueuse contenant du sel pour ainsi former une couche d'exsiccation qui stabilise et protège la surface de l'implant, ledit implant étant constitué d'un matériau qui est choisi dans le groupe des métaux, des alliages métalliques et de leurs combinaisons avec des matériaux céramiques et qui est optionnellement pourvu d'une couche d'oxyde sur sa surface.

2. Procédé selon la revendication 1, la solution dans laquelle on introduit ledit implant étant une solution aqueuse contenant du sel qui est inerte vis-à-vis de la surface ultrahydrophile et qui présente une concentration totale d'ions supérieure à 0,5 mol/L, de préférence supérieure à 1 mol/L.

3. Procédé selon la revendication 1 ou 2, la solution aqueuse contenant du sel étant une solution d'un sel unique ou d'une combinaison de différents sels dans l'eau, le sel étant préférentiellement choisi dans le groupe constitué de l'ensemble des sels dont l'anion est parmi SO₄⁻⁻, HPO₄⁻⁻, H₂PO₄⁻, CH₃COO⁻, Cl, Br⁻, NO4-, ClO₄⁻, I⁻, CNS⁻, ClCH₂COO⁻, F₃CCOO⁻, Cl₂CHCOO⁻, Cl₃CCOO⁻, Br₃CCOO⁻ ou dont le cation est parmi NH₄⁺, Rb⁺, K⁺, Na⁺, Cs⁺, Li⁺, Mg⁺⁺, Ca⁺⁺, Ba⁺⁺ ainsi que (CH₃)₄N⁺, (C₂H₅)₄N⁺, (C₃H₇)₄N⁺, (C₄H₉)₄N⁺.

4. Procédé selon l'une des revendications 1 à 3, comportant l'utilisation de la solution contenant du sel dans une quantité et à une concentration de sel qui sont telles que, suite à l'évaporation, on obtient une couche d'exsiccation recouvrant au moins la surface ultrahydrophile dudit implant avec une épaisseur de couche comprise entre 1 et 500 µm.

5. Procédé selon l'une des revendications 1 à 4, comportant l'utilisation d'une solution aqueuse contenant du sel laquelle donne, suite à l'évaporation, une couche d'exsiccation entourant ledit implant de tous les côtés.

6. Procédé selon l'une des revendications 1 à 5, comprenant l'étape supplémentaire consistant à stériliser ledit implant.

7. Procédé selon la revendication 6, la stérilisation dudit implant comprenant une stérilisation à l'aide d'un rayonnement ionisant, notamment d'un rayonnement électromagnétique.

8. Implant apte au stockage lequel a été obtenu conformément au procédé selon l'une des revendications 1 à 7 ou lequel comporte une surface ultrahydrophile qui est revêtue d'une couche d'exsiccation.

9. Implant apte au stockage selon la revendication 8, ledit implant étant constitué d'un matériau choisi dans le groupe des métaux, des alliages métalliques et de leurs combinaisons avec des matériaux céramiques et comportant, optionnellement, une couche d'oxyde sur sa surface.

10. Implant apte au stockage selon les revendications 8 à 9, le matériau d'implant mis en œuvre correspondant à des matériaux métalliques tels que le titane pur ou les alliages métalliques du titane, les alliages de chrome/nickel/aluminium/vanadium/cobalt, le TiAlV4, le TiAlFe2,5, les aciers inoxydables, le V2A, le V4A, le chrome-nickel 316L ou à une de leurs combinaisons avec des matériaux céramiques tels que l'hydroxyapatite, l'oxyde d'aluminium dans lesquelles le matériau métallique se présente sous forme d'un matériau composite avec des matériaux céramiques.

11. Implant apte au stockage selon l'une des revendications 8 à 10, la surface ultrahydrophile présentant une hystérésis de l'angle de contact inférieure à 5° lorsque la surface de l'implant est mouillée avec de l'eau.

12. Implant apte au stockage selon l'une des revendications 8 à 11, la couche d'exsiccation étant préparée en introduisant ledit implant dans une solution aqueuse contenant du sel, de préférence dans une solution aqueuse de sel neutre qui est inerte vis-à-vis de la surface ultrahydrophile et qui vient entourer l'implant de tous les côtés, et en évaporant à siccité la solution aqueuse contenant du sel.

13. Implant apte au stockage selon la revendications 12, la couche d'exsiccation étant préparée en introduisant ledit implant dans une solution aqueuse contenant du sel, de préférence dans une solution aqueuse de sel neutre, laquelle a une concentration totale d'ions supérieure à 0,5 mol/L.

14. Implant apte au stockage selon l'une des revendications 8 à 13, la couche d'exsiccation étant constituée d'un seul sel ou d'une combinaison de différents sels, le sel étant préférentiellement choisi dans le groupe constitué de l'ensemble des sels dont l'anion est parmi SO₄⁻⁻, HPO₄⁻⁻, H₂PO₄⁻, CH₃COO⁻, Cl, Br⁻, NO4-,ClO₄⁻, I⁻, CNS⁻, ClCH₂COO⁻, F₃CCOO⁻, Cl₂CHCOO⁻, Cl₃CCOO⁻, Br₃CCOO⁻ ou dont le cation est parmi NH₄⁺, Rb⁺, K⁺, Na⁺, Cs⁺, Li⁺, Mg⁺⁺, Ca⁺⁺, Ba⁺⁺ ainsi que (CH₃)₄N⁺, (C₂H₅)₄N⁺, (C₃H₇)₄N⁺, (C₄H₉)₄N⁺.
